(19) Europäisches Patentamt
European Patent Office
Office européen des brevets

(11) EP 2 968 134 B1

# (12) EUROPEAN PATENT SPECIFICATION

(45) Date of publication and mention of the grant of the patent:
**01.09.2021 Bulletin 2021/35**

(21) Application number: **14769929.2**

(22) Date of filing: **13.03.2014**

(51) Int Cl.:
***A61K 9/08*** (2006.01) ***A61K 47/10*** (2017.01)
***A61K 47/30*** (2006.01) ***A61K 47/02*** (2006.01)
***A61K 9/10*** (2006.01)

(86) International application number:
**PCT/US2014/025782**

(87) International publication number:
**WO 2014/151459 (25.09.2014 Gazette 2014/39)**

## (54) AQUEOUS DELIVERY SYSTEM FOR LOW SURFACE ENERGY STRUCTURES

WÄSSRIGES ABGABESYSTEM FÜR STRUKTUREN MIT GERINGER OBERFLÄCHENENERGIE

SYSTÈME D'ADMINISTRATION AQUEUSE POUR DES STRUCTURES À FAIBLE ÉNERGIE DE SURFACE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201313837243**

(43) Date of publication of application:
**20.01.2016 Bulletin 2016/03**

(73) Proprietor: **W. L. Gore & Associates, Inc.**
**Newark, DE 19711 (US)**

(72) Inventors:
- **FREESE, Donald, T.**
**Waikoloa, HI 96738-4704 (US)**
- **EDMUNDSON, Mark, D.**
**Wilmington, DE 19806 (US)**

(74) Representative: **HGF**
**1 City Walk**
**Leeds LS11 9DX (GB)**

(56) References cited:
**WO-A1-00/66655** **WO-A1-2012/135895**
**WO-A2-2006/127937** **WO-A2-2006/127946**
**US-A1- 2006 270 744** **US-B1- 6 228 477**
**US-B1- 6 235 662**

- **None**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 968 134 B1

## Description

### FIELD OF THE INVENTION

The present invention relates generally to an aqueous system for coating low surface energy surfaces and coated surfaces formed therefrom, and more particularly, to filling low surface energy microporous materials with inorganic particles and products formed therefrom.

### BACKGROUND OF THE INVENTION

Conventional aqueous micellar delivery systems have been used predominantly in the pharmaceutical industry to provide both controlled delivery of drugs and controlled release of pharmaceutical agents. A micellar solution is one that contains at least one surfactant at a concentration greater than the critical micelle concentration ("CMC"). In the case of aqueous micellar solutions, when a hydrophobic or less water soluble material such as an oil is emulsified in the micellar solution, an emulsion results. Due to the often high surfactant concentrations used in many emulsions, the resulting surfactant stabilized emulsion droplets are often very stable. The good stability against coalescence also makes emulsion droplets ideal carriers for other materials. This technology is typically used in the pharmaceutical industry for controlled delivery of pharmaceutical agents such as antibiotics, antimicrobials, antivirals, cardiovascular and renal agents. These agents are commonly incorporated into the hydrophobic component of the carrier emulsion. Frequently, such emulsions are comprised of a hydrophobic material selected from the group consisting of a long chain carboxylic acid, long chain carboxylic acid ester, long chain carboxylic acid alcohol and mixtures thereof.

A permutation of these aqueous micellar delivery systems are microemulsions which form easily, even spontaneously, in the presence of typically high emulsifier concentrations. Microemulsions are particularly useful as delivery vehicles because a range of materials can be contained therein that would otherwise be sensitive to water, such as hydrolysis sensitive materials. In typical pharmaceutical microemulsion applications, the hydrophobic material is a water insoluble organic material that is emulsified by surfactants to form a discontinuous phase in a continuous aqueous phase (see, for example, U.S. Patent No. 5,952,004 to Rudnic, et.al.). Such microemulsions can be extremely stable and can provide a useful delivery means. For example, pharmaceutical agents may be dispersed into the hydrophobic material and delivered as part of the aqueous emulsion.

Emulsion technology is also used to create polymeric dispersions wherein a monomer is first emulsified in an aqueous surfactant solution and then polymerized. The resulting emulsion polymers, commonly referred to as latexes, have found many uses including paints and coatings. In order for a latex to spread across the substrate surface and form a uniform coating, it is necessary for it to "wet" the substrate to which it is applied. "Wetting" results when the contact angle, $\theta$, between the aqueous latex and the solid substrate is less than about 90 degrees. Spontaneous wetting occurs when the surface energy between the solid and liquid, $\gamma SL$ is less than the surface energy between the solid and air, $\gamma SA$. The relationship between these parameters and the liquid-air surface energy, yLA, is given by the relationship below:

$$ySL = ySA - yLA * \cos(\theta)$$

This relationship is very important when trying to coat a low surface energy substrate (low ySA), such as for example, materials with a surface energy below about 40 dynes/cm, because a very low ySL is required.

One low surface energy substrate of particular interest is polytetrafluoroethylene ("PTFE") and microporous polytetrafluoroethylene. Due to the inherent hydrophobicity of PTFE, membranes of these materials are of particular interest when in the form of repellent products such as rainwear. Expanded microporous, liquid waterproof polytetrafluoroethylene materials, such as those available from W.L. Gore and Associates, Inc., sold under the trademark GORE-TEX®, as well as expanded PTFE products available from other suppliers are especially well suited for this purpose. The expanded PTFE materials are liquid waterproof, but allow water vapor, in the form of perspiration, to pass through. Polyurethanes and other polymers have been used for this purpose also. To confer good flexibility and light weight in the materials for use in the textile sector, the microporous layer should be made as thin as possible. However, a thinner membrane will generally mean a loss of performance, and thin coatings run the risk of decreasing water repellency.

Low surface energy substrates have historically been coated by solutions having a low yLA and low contact angle. Suitable coating processes for microporous low surface energy materials are described in the art, many of which rely on solvents to wet the desired substrate. For example, EP 0581168 (Mitsubishi) describes the use of perfluoroalkyl methacrylates and perfluoroalkylethyl acrylates for porous polyethylene and polypropylene membranes. These substances are held in physical contact with the surface of the polyolefin porous membrane. The fluorinated monomer or fluorinated monomer and a crosslinking monomer together with a polymerization initiator are dissolved in a suitable solvent to prepare a solution. For example this solution typically can comprise 15% wt. monomer and 85% wt. acetone.

After coating, the solvent is vaporized off. The situation is similar to a process for treating the surfaces of polymers with essentially pure solvent solutions containing low concentrations (e.g. less than 1.0% wt.) of amorphous fluoropolymers (WO 92/10532). Likewise, solutions of fluorine-containing polymers are also involved in a patent for coating ePTFE with an amorphous copolymer of tetrafluoroethylene (EP 0561875). In each of these cases, significant quantities of solvent are released during the coating coalescence process. These solvent emissions are both costly and environmentally undesirable. Moreover, solvent-based wetting systems have the inherent limitation of incompatibility with a broad range of aqueous fluoropolymers, and the concentration of solvent necessary to wet the substrate limits the amount and type of additive that can be coated on that substrate.

Efforts have been made to convert from these solvent-based coating systems to aqueous coatings systems. However, the challenges of achieving stability of the wetting package and achieving fast wetting speed are hard to meet. One relatively common approach is to add a water soluble organic solvent to the aqueous coating solution or latex. U.S. Patent No. 6,228,477 teaches a means to coat a low surface energy, microporous PTFE substrate with an otherwise non-wetting, aqueous fluoropolymer dispersion through the use of significant percentages of isopropanol ("IPA"). In one such example, the non-wetting, aqueous fluoropolymer dispersion was diluted to 25% dispersion and 75% IPA, applied to a microporous PTFE substrate, and the solvent evaporated off to thereby form a uniform coating of the desired fluoropolymer. This process unfortunately requires the use of large amounts of IPA and creates significant environmental problems. In other examples in this patent, a number of fluoropolymer treatments were shown to be unstable with high concentrations of water soluble alcohol, further limiting this IPA wetting system. Further examples of an aqueous delivery system is described in U.S. Patent Application No. 2006/0270744 to Freese, International patent application no. WO 2006/127937 to Freese, and International patent application no. WO 2012/135895 to R.J. Roberts Consulting PTY Ltd.

Aqueous microemulsion systems have been developed to circumvent the need for high levels of VOC's in order to wet low surface energy substrates. One such system that does not require the use of IPA or any other VOC's is taught in U.S. Patent No. 5,460,872, to Wu et.al. This patent teaches the use of fluorinated surfactants to lower the surface energy and contact angle with microporous PTFE as a means to produce a uniformly coated microporous PTFE substrate. After application of this aqueous dispersion, the fluorinated surfactant and the residual water were then removed by heating.

High costs of manufacturing and potential environmental issues with these prior art materials have highlighted the continuing need for a solution to effectively coat low surface energy substrates without high levels of VOC's or undesirable fluorosurfactants.

## SUMMARY OF THE INVENTION

The present invention as defined in the appended claims relates to a robust, stable aqueous delivery system. The invention is capable of wetting low surface energy substrates and thereby can deliver a wide range of organic and inorganic materials to form coatings thereon. Additionally, the stable aqueous delivery system can be used to fill the pores of low surface energy microporous substrates with inorganic particles. The present invention is directed, at least in part, to an aqueous delivery system of a surfactant and a water insoluble alcohol wetting agent. Optionally, one or more materials that permit greater amounts of wetting agent without causing phase separation (i.e., stabilizers) can be added. Added functionality can be incorporated by including one or more additives in the aqueous delivery system. Inorganic particles and optionally dispersants can be included in the aqueous delivery system. The inventive aqueous delivery system may be used to deliver a range of functional materials such as, for example, functionalized or surface active polymers and inorganic particles to low surface energy materials. Also described are low surface energy materials, such as microporous fluoropolymers, coated by the aqueous delivery system. Additionally, the invention includes a coated article including a low surface energy microporous material having a coating on at least a portion of the pore walls of the microporous material where the coating has a measurable amount of surfactant and a water insoluble alcohol in an amount up to 25% by weight based on the total weight of the coated microporous material. Further, the invention includes a low surface energy microporous material whose pores are substantially or completely filled with the elements found in Group 1A, IIA, 118, IVB, VB, VIB, VIIB, VIII, IB, IIB, IIIA, IVA; VA, and VIA of the periodic table; metal oxides or hydroxides; metal nitrides; silicates; carbonates; sulfonates; phosphates; nitrates; and mixtures thereof.

## DESCRIPTION OF FIGURES

Figure 1 is a scanning electron micrograph (SEM) of a cross section of the article prepared in Example 11.
Figure 2 is a scanning electron micrograph (SEM) of a cross section of the article prepared in Example 13.
Figure 3 is a scanning electron micrograph (SEM) of a cross section of the article prepared in Example 17.

## DETAILED DESCRIPTION OF INVENTION

In the present invention, an aqueous solution is produced when at least one surfactant is used to emulsify at least one water insoluble wetting agent. In a further embodiment, this invention is directed to low surface energy substrates, such as microporous polytetrafluoroethylene, coated with such an aqueous solution so as to impart a change in at least one surface characteristic compared to the surface characteristics of the uncoated microporous substrate.

Application to low surface energy substrates relies on good wetting. To achieve good wetting, the surface tension of the aqueous delivery system should be sufficiently low to penetrate the microporous substrate. For example, a surface tension of less than or equal to about 0.03 N/m (30 dynes/cm) is typically required to penetrate expanded microporous PTFE. Higher surface tension wetting systems may accordingly be suitable for higher energy substrates such as microporous polyethylene or microporous polypropylene. As previously discussed, the prior art teaches that high levels of water soluble wetting agents such as isopropanol can be used to lower $\gamma SL$ in order to enable certain aqueous coating systems to wet a microporous low surface energy PTFE substrate (see, e.g. U.S. Pat. No. 6,676,993 B2).

Suitable wetting agents of the present invention include alcohols and mixtures of alcohols that exhibit a low water solubility, such as those alcohols having five or more carbon atoms in the longest continuous chain, e.g., alcohols with $C_5$-$C_{10}$ linear chains, and the like. For example, pentanols, hexanols, octanols, and the like, are within the range of suitable wetting agents of the present invention. Further, the aqueous delivery system can incorporate with the water insoluble alcohol(s) other water insoluble organics, such as alkanes, etc. Optionally, the wetting agent may also exhibit a low $\gamma SL$ relative to the targeted low surface energy substrate.

The surfactant(s) of this invention can be a single surfactant or a combination of surfactants. Suitable surfactants are defined as those that are able to emulsify the desired wetting agent. For the alcohols described above, several classes of anionic surfactants used may be those having a structure of $R(EO)nOSO_3^-$ or $ROSO_3^-$ where R can be any organic chain, "O" is oxygen, "S" is sulfur, "EO" is ethylene oxide and n=>1. In an alternate embodiment, nonionic surfactants having the structure $R(EO)_nOH$, where n=>1, are also suitable for this invention. In at least one exemplary embodiment, nonionic surfactants with a hydrophilic-lipophilic balance ("HLB") value of ten or greater were found most effective to emulsify the wetting agents described above. The concentration of surfactant can be adjusted in order to achieve good emulsification of the desired wetting agent. For example, when 4% by weight of hexanol wetting agent (based on the total aqueous solution weight) is used, a concentration of about 2% of sodium dodecyl ether sulfate was found to be suitable. In an alternate formulation, 6% wt. of an ethoxylated alcohol was able to emulsify 4% wt. hexanol wetting agent.

In addition to the aqueous delivery system provided by the surfactant and the wetting agent, a stabilizing agent can optionally be added. A stabilizing agent is typically soluble in both the alcohol and water, and it allows a greater amount of alcohol to be stabilized in the aqueous system than without the stabilizer. In one embodiment, glycols were found to be effective stabilizers, such as, but not limited to, dipropylene glycol ("DPG"), dipropylene glycol monomethyl ether, and propylene glycol. A wide range of stabilizer concentrations can be used depending on the amount of additional stability desired. For instance, if a small increase in stability is desired, a small amount of the optional stabilizer should be used. Conversely, higher stabilizer concentrations generally further increase the emulsion stability. Exceptions to these general guidelines do however exist. For example, DPG may be an effective stabilizer when used in concentrations ranging from less than about 1% wt. up to about 10% wt. based on total aqueous emulsion weight for hexanol-based systems.

In another aspect of this invention, additional functional additives can optionally be added to the aqueous delivery system. As used herein, the term "functional additive" is intended to refer to any additional material which renders further functionality to the low surface energy substrate than what otherwise exists in the absence of the functional additive. Suitable functional additives include materials which have suitable stability to be delivered and which are either soluble in the aqueous delivery system (either the water or wetting agent) or dispersable in the aqueous delivery system. In one exemplary embodiment of the invention, if the substrate is a polymer layer that is not naturally oleophobic, it can be rendered oleophobic by incorporating within the aqueous delivery system a functional additive which is an oleophobic material. This unique feature of the invention provides significant advantages over conventional solvent coating means of applying, for example, oleophobic materials. This unique delivery system of the present invention provides spontaneous wetting of the substrate, and even in the case of microporous substrates, such as described below, which often have tortuous porosity, the present invention can be tailored to readily facilitate coating at least a portion of the pore walls of the substrate.

In one embodiment of this invention, suitable low surface energy materials are microporous substrates, as noted in the previous paragraph. Suitable microporous polymers can include fluoropolymers (e.g. polytetrafluoroethylene or polyvinylidene fluorides), polyolefins (e.g. polyethylene or polypropylene); polyamides; polyesters; polysulfone, poly(ethersulfone) and combinations thereof, polycarbonate, and polyurethanes. Coatings applied via the present invention to such microporous substrates may be designed to either coat the surfaces of the microstructure leaving the pores open or they can be designed to effectively fill a substantial portion of the pores. In instances where retention of air

permeability or high breathability is desired, the present invention should be designed to preserve the open microporous structure, as filling the micropores may destroy or severely lessen the water-vapor transmitting property of the microporous substrate. Thus, the walls defining the voids in the microporous polymer desirably have only a very thin coating of the oleophobic polymer in such an embodiment. Moreover, to maintain flexibility of the substrate, the coating of the functional material should be sufficiently thin so as not impact the flexibility of the substrate when coated.

Common oleophobic functional additive compositions suitable for this invention include oleophobic fluorocarbon compounds. For example, the fluorocarbon can be one that contains perfluoroalkyl groups $CF_3$ --$(CF_2)_n$--, where n is ≥0. The following compounds or classes of oleophobic materials, while not exhaustive, can be used:

- Apolar perfluoropolyethers having $CF_3$ side groups, such as Fomblin YAusimont; Krytox--DuPont;
- Mixtures of apolar perfluoroethers with polar monofunctional perfluoropolyethers PFPE (Fomblin and Galden MF grades available from Ausimont);
- Polar water-insoluble PFPE such as, for example, Galden MF with phosphate, silane, or amide, end groups;
- Mixtures of apolar PFPE with fluorinated alkyl methacrylates and fluorinated alkyl acrylate as monomer or in polymer form.

The above-mentioned compounds can also optionally be crosslinked by, for example, UV radiation in aqueous form solution or emulsion.

The following polymeric particle solutions, while again not exhaustive, can also be used:

- Microemulsions based on PFPE (see EP 0615779, Fomblin Fe20 microemulsions);
- Emulsions based on copolymers of siloxanes and perfluoroalkyl- substituted (meth)acrylates (Hoechst);
- Emulsions based on perfluorinated or partially fluorinated co- or terpolymers, one component containing at least hexafluoropropene or perfluoroalkyl vinyl ether;
- Emulsions based on perfluoroalkyl-substituted poly(meth)acrylates and copolymers (products of Asahi Glass, Hoechst, DuPont and others).
- Microemulsions based on perfluoroalkyl-substituted poly(meth)acrylates and copolymers (U.S. Pat. No. 5,539,072 and U.S. Pat. No. 5,460,872);

The concentration of the functional material provided by this invention can vary greatly depending on the desired outcome. When an oleophobic fluoropolymer is used as the functional additive material, such as, but not limited to, polymers having - -$(CF_2)_n$--$CF_3$ pendant groups, functional materials of this type can impart very low surface energy values to the polymer and thus impart good oil and water resistance properties. Representative oleophobic polymers can be made from organic monomers having pendant perfluoroalkyl groups. These include fluoroalkyl acrylates and fluoroalkyl methacrylates having terminal perfluoroalkyl groups of the formula:

$$CF_3(CF_2)_n - (CH_2)_m - O - \overset{\overset{\displaystyle O}{\|}}{C} - \underset{\underset{\displaystyle R}{|}}{C} == CH_2$$

wherein n is a cardinal number of 1-21, m is a cardinal number of 1-10, and R is H or $CH_3$; fluoroalkyl aryl urethanes, fluoroalkyl allyl urethanes, fluoroalkyl urethane acrylates; fluoroalkyl acrylamides; fluoroalkyl sulfonamide acrylates and the like. When a low surface energy coating is desired, concentrations from about 1 % wt. up to about 20% wt. based on total emulsion solids may be effective. When coating microporous substrates, the concentration of the oleophobic functional material is desirably between about 3% wt. up to about 12% wt. based on total emulsion weight.

Alternate embodiments of this invention include other optional functional additive materials. The present invention can be used to deliver particulate functional materials to surfaces, provided that the particulate can be dispersed in the emulsion wetting system. In some instances, it may be advantageous to disperse the particulates in a dispersing agent which can subsequently be dispersed in the emulsion wetting system. Hence when a substrate is coated with the aqueous solution, the functional additive particles contained therein will be deposited onto and/or into the substrate and its surfaces in order to effect, for example, a color change in the case of a pigment, or other desirable functional change in the substrate. Carbon particles are of particular interest in applications where a change in an electromagnetic spectral

response or electric or thermal conductivity of the substrate is desired. Particulate concentrations of 10%, 15%, 20%, 25%, 30%, 35%, 40% or even higher may be utilized to fill or substantially fill the pores of the microporous substrate.

The optional functional material of the present invention may also be materials that are either soluble in the inventive aqueous delivery system. The list of soluble materials that can be used in conjunction with the present invention include, but are not limited to, simple salts (e.g., $AgNO_3$, $CuSO_4$), simple compounds, polyacrylic acid, polyacrylamide, melamine, polyvinyl alcohol, salts, and dyes. The list of dispersible materials that can be used in conjunction with the present invention include, but are not limited to, polyfluoroacrylates, polystyrene, pigments, carbon black, and aluminum oxide, as well as other insoluble compounds or inorganic particles. The inorganic particles comprise water insoluble compounds or elements selected from the group consisting of metals; carbon; metal oxides or hydroxides; metal nitrides; silicates; carbonates; sultanates; phosphates; nitrates; or mixtures thereof. The aqueous delivery system in these embodiments can optionally include other components including, but not limited to, a variety of dispersants, stabilizers and de-foaming agents that are known in the art to produce stable, fluid dispersions of solids in liquids. Particularly preferable dispersants in these embodiments include sultanate co-polymers, maleic anhydride co-polymers, and active polymeric dispersants in water designed for water-based paint (automotive & industrial) and water-based inks.

One requirement for the dispersible materials is that the particle size be sufficiently small so that the materials can physically enter the pores of the rnicroporous substrate to which they are being applied. When the microporous substrate is inherently hydrophobic, such a coating can change the surface characteristic from hydrophobic to hydrophilic. Further, the dispersion solution has surprisingly been found to wet an expanded polytetrafluoroethylene membrane sufficiently to uniformly fill at least a portion of the pores through the thickness of the expanded polytetrafluoroethylene membrane.

In order to enhance the filling of the pores with inorganic particles, various processes known in the art may be used to reduce the particle size and/or break up agglomerates of particles within the inventive aqueous delivery system. Generally, these methods apply some form of mechanical energy to the solution using sound waves or other mechanical means. Such methods include, but are not limited to, high shear mixers known in the art such as wet jet mills (e.g., a Microfluidizer by Microfluidics of Newton, MA), or rotor-stator mixers comprising at least one stage. When the high shear mixer is a Microfluidizer, it may operate at a pressure between about 1,000 and about 25,000 psi. Other methods to reduce agglomeration in the dispersion may also be used, including, but not limited to, ultrasonic baths or horns. Use of such processes also enhances the ability of the dispersions to be fluid. As used herein, fluid means that a solution has a viscosity low enough so it readily pours and easily spreads across a microporous substrate.

One surprising result of the instant invention is the ability to substantially uniformly fill or uniformly fill at least a portion of the pores of a microporous substrate, such as, for example, an expanded polytetrafluoroethylene substrate. Even more surprising is how high a concentration of the particles in the final article can be achieved, with concentrations up to 98 weight percent of the inorganic particles in the final article. It is also possible to fill the pores of the microporous substrate partially by controlling the particle concentration in the aqueous delivery system. Thus, one can fill the pores with inorganic particles in concentrations to any amount between zero and 98 weight percent, for example, up to about 5, about 10, about 20, about 30, about 50, or about 75 weight percent, or more of the final article. In these embodiments of the invention, the concentration is uniform through the thickness of the article. As used herein, uniformly filling a microporous substrate means that a micrograph, for example a scanning electron micrograph (SEM), of a cross-section of a filled microporous substrate shows no distinct boundary between layers of filled pores and un-filled pores between the top and bottom surfaces of the microporous substrate, nor does it visually show any clear gradient in particle concentration in the micrograph between the top and bottom surfaces of the microporous substrate.

Other useful permutations of this invention are also encompassed within the breadth of functional materials that can be stable in the present aqueous delivery system and thereby subsequently applied to a range of microporous and nonmicroporous substrates as defined in the claims.

## DEFINITIONS

For the purposes of this application the following terms shall be recognized to have the meaning set forth below unless otherwise indicated:

"Air permeable" means that airflow is observed as determined by the Gurley test described below. It will be appreciated by one of skill in the art that an air permeable material will also be moisture vapor permeable.

"Air-impermeable" means that no airflow is observed for at least two minutes as determined by the Gurley test described below.

"Hydrophilic" material denotes a porous material whose pores become filled with liquid water when subjected to liquid water without the application of pressure.

"Microporous" term is used to denote a continuous layer of material comprised of interconnecting pores which create a passageway extending from one surface of the layer to the opposite surface of the layer.

"Oleophobic" means a material that has an oil resistance of 1 or more, as measured by the Oil Repellency Test,

below.

## TEST PROCEDURES

### Air Permeability/impermeability--Gurley Number Test

Gurley numbers were obtained as follows:
The resistance of samples to air flow was measured by a Gurley densometer (ASTM 0726-58) manufactured by W. & L. E. Gurley & Sons. The results are reported in terms of Gurley Number, which is the time in seconds for 100 cubic centimeters of air to pass through 6.54 cm. sup.2 of a test sample at a pressure drop of 1.215 kN/$m^2$ of water. A material is air-impermeable if no air passage is observed over a 120 second interval.

### Oil Repellency Test

In these tests, oil rating was measured using the AATCC Test Method 118-1983 when testing film composites. The oil rating of a film composite is the lower of the two ratings obtained when testing the two sides of the composite. The higher the number, the better the oil repellency. A value of greater than 1, preferably 2 or more, more preferably 4 or more, is preferred.

The test is modified as follows when testing laminates of the film composite with a textile. Three drops of the test oil are placed on the textile surface. A glass plate is placed directly on top of the oil drops. After 3 minutes, the reverse side of the laminate is inspected for a change in appearance indicating penetration or staining by the test oil. The oil rating of the laminate corresponds to the highest number oil that does not wet through the laminate or cause visible staining from the reverse side of oil exposure. The higher the number, the better the oil repellency. A value of greater than 1, preferably 2 or more, more preferably 4 or more, and most preferably, 6 or more, is preferred.

## EXAMPLES

### Example 1 (not falling within the scope of the invention)

In order to determine the amount of 1-hexanol needed to wet ePTFE with a non-ionic surfactant, a nonionic surfactant, Iconol DA-6 (BASF, ethoxylated alcohol, HLB 13), was added to de-ionized water to make a 4 weight % solution. 1-Hexanol was added incrementally to the Iconol DA-6 solution. After each addition of 1-hexanol, the stability of the mixture was examined for phase separation.

The ability of this mixture to wet and penetrate a 50 g/m2 ePTFE membrane (0.2 micron pore size, 100 micron thickness, Gurley number of about 25 sec., W. L.Gore and Associates, Inc., Elkton, MD) was assessed by measuring the time required for a drop to clarify fully the membrane. The data are shown in Table I. Pure 1-hexanol wets ePTFE in 1-2 sec. Surprisingly, a dilute hexanol (1.7%) and surfactant blend wets ePTFE as fast as pure hexanol.

**Table I**

| Weight % 1-Hexanol | Time to Clarify ePTFE (sec) | Stability |
|---|---|---|
| 0 | >30 | stable, 1 phase |
| 1.2 | 4 | stable, 1 phase |
| 1.7 | 1-2 | stable, 1 phase |

### Example 2 (not falling within the scope of the invention)

Witcolate ES-2 (30% solids, dodecyl ether sulfate, obtained from Witco Chemicals/Crompton Corporation, Middlebury, CT) was used to determine that a level as high as approximately 11 % surfactant solids could be used to wet ePTFE (50 g/m2) in combination with 1-hexanol. A mixture of 3.9 g of Witcolate ES-2 and 6.1 g of de-ionized water was prepared. 1-Hexanol was added incrementally to this mixture, and the stability and wetting time for ePTFE was measured as in Example 1. The data are shown in Table II.

**Table II**

| Witcolate ES-2 (Wt% solids) | Time to Clarify 1-Hexanol (Wt%) | ePTFE (sec) | Stability |
|---|---|---|---|
| 12 | 0 | >30 | stable, 1 phase |
| 12 | 1.4 | >30 | stable, 1 phase |

(continued)

| Witcolate ES-2 (Wt% solids) | Time to Clarify 1-Hexanol (Wt%) | ePTFE (sec) | Stability |
|---|---|---|---|
| 11 | 3.7 | >30 | stable, 1 phase |
| 11 | 5.9 | partial in 30 sec | stable, 1 phase |
| 11 | 7.6 | 7 | stable, 1 phase |
| 11 | 8.5 | >30 | stable, 1 phase |

**Example 3 (not falling within the scope of the invention)**

In order to determine the upper range of 1-hexanol (approximately 30 weight %) that could be used to wet ePTFE, 1.3 g of Witcolate ES-2 (30% solids, dodecyl ether sulfate, obtained from Witco Chemicals/Crompton Corporation, Middlebury, CT) was added to 8.7 g of de-ionized water. 1-Hexanol was added incrementally to this mixture, and the stability and wetting time for ePTFE was measured as in Example 1. The data are shown in Table III.

**Table III**

| Witcolate ES-2 (Wt% solids) | 1-Hexanol (Wt%) | Time to Clarify ePTFE (sec) | Stability |
|---|---|---|---|
| 4 | 0 | >30 | stable, 1 phase |
| 3 | 13 | 2 | stable, 1 phase |
| 3 | 17 | 2 | stable, 1 phase |
| 3 | 21 | 3 | stable, 1 phase |
| 3 | 25 | 2 | stable, 1 phase |
| 3 | 31 | 10 | stable, 1 phase |

**Example 4 (not falling within the scope of the invention)**

In addition to nonionic and anionic surfactants, cationic surfactants were determined to be useful in combination with 1-hexanol to wet quickly ePTFE, as follows. A dodecyldimethylethyl quaternary ammonium bromide, DAB, (0.3 g) was added to 9.7 g of de-ionized water. 1-Hexanol was added incrementally to this mixture, and the stability and wetting time for ePTFE was measured as in Example 1. The data are shown in Table IV.

**Table IV**

| DAB (weight%) | 1-Hexanol (Weight %) | Time to Clarify ePTFE (sec) | Stability |
|---|---|---|---|
| 3 | 0 | >30 | stable, 1 phase |
| 3 | 1.3 | >30 | stable, 1 phase |
| 3 | 2.7 | 13 | stable, 1 phase |
| 3 | 4.2 | 2 | stable, 1 phase |

**Example 5 (not falling within the scope of the invention)**

To determine that compounds soluble in both the water-insoluble alcohol and water such as dipropylene glycol (DPG) can be used to increase the stability of the wetting mixture, a mixture (4 weight%) of a nonionic ethoxylated alcohol surfactant (Iconol TDA-9 from BASF) was prepared. Without DPG, 1-hexanol would cause phase separation at 2.5 % 1-hexanol. The addition of 4 weight % DPG increased the stability and the ability to wet ePTFE (50 g/m2). The data are shown in Table V.

**Table V**

| Iconol TDA-9 (Wt.%) | 1-Hexanol (Wt.%) | DPG (Wt.%) | Time to Clarify ePTFE (sec) | Stability |
|---|---|---|---|---|
| 4 | 0 | 4 | >30 | stable, 1 phase |
| 4 | 0 | 4 | 15 | stable, 1 phase |
| 4 | 2.1 | 4 | 12 | stable, 1 phase |
| 4 | 2.9 | 4 | 7 | stable, 1 phase |
| 4 | 3.6 | 4 | <1 | stable, 1 phase |

**Example 6 (not falling within the scope of the invention)**

Other water-insoluble alcohols were also examined. Pure 1-octanol clarifies ePTFE (50 g/m2) in 5 seconds. A dilute mixture of 1-octanol with Witcolate ES-2 (30% solids, dodecyl ether sulfate, obtained from Witco Chemicals/Crompton Corporation, Middlebury, CT) can wet ePTFE as fast as pure octanol. A 13 weight percent (4 weight% solids) Witcolate ES-2 solution was prepared. 1-Octanol was added incrementally to this mixture. The stability and wetting time for ePTFE was measured as in Example 1. The data are shown in Table VI.

**Table VI**

| Witcolate ES-2 (Wt% solids) | 1-Octanol (Wt %) | Time to Clarify ePTFE (sec) | Stability |
|---|---|---|---|
| 4 | 0 | >30 | stable, 1 phase |
| 4 | 1.4 | >30 | stable, 1 phase |
| 4 | 2.9 | 21 | stable, 1 phase |
| 4 | 3.9 | 11 | stable, 1 phase |
| 4 | 4.9 | 7 | stable, 1 phase |
| 4 | 6.2 | 5 | stable, 1 phase |
| 4 | 7.3 | 4 | stable, 1 phase |

**Example 7 (not falling within the scope of the invention)**

The ability of surfactant and hexanol mixtures to wet and coat ePTFE with oleophobic materials was examined. Mixtures of 13 weight percent (4 weight percent solids) Witcolate ES-2 (30% solids, dodecyl ether sulfate, obtained from Witco Chemicals/Crompton Corporation, Middlebury, CT) and approximately 6 weight percent 1-hexanol were prepared with various fluoroacrylate polymers (9 weight percent solids). The following fluoropolymers were used: AG415 and AG4210 (Asahi Glass Company), Zonyl 7040 (DuPont), and TG-532 (Daikan). These mixtures were spread on one surface of an expanded PTFE membrane (about 20 g/m2, thickness of about 40 micron, and Gurley number of about 15 sec.) until the membrane was clarified. The coated ePTFE was placed in a solvent oven at 190°C for 2.5 min. The time for a drop of these coating mixtures to clarify ePTFE (50 g/m2) was measured. The stability of the mixture was examined. Oil ratings on the coated and uncoated side of the ePTFE (20 g/m2) were measured. Additionally, the air permeability was determined by measuring the time for 100 cm3 of air to flow through the coated membrane (Gurley). The data show that a range of fluoropolymers can be used to coat ePTFE (Table VII). The uncoated ePTFE has a Gurley of 15.7 sec. The oil rating of ePTFE (uncoated) was 1.

**Table VII**

| Witcolate ES-2 (wt% solids) | 1-Hexanol (wt%) | Fluoropolymer Type / (wt% solids) | Time to Wet ePTFE (sec) | Oil Rating (coated / uncoated side) | Gurley (sec) |
|---|---|---|---|---|---|
| 3.9 | 6.1 | AG415/9 wt% | 2 | 8/6 | 62.9 |
| 3.9 | 6.3 | Zonyl 7040/9 wt% | 2 | 7/6 | 57.3 |
| 3.9 | 6.1 | TG532/9 wt% | 1 | 8/8 | 38.1 |
| 3.9 | 5.0 | AG4210/9 wt% | | 8/7 | 38.7 |

**Example 8**

Multiple functional additives were used to coat an expanded PTFE membrane (20 g/m2, W. L. Gore and Associates, Inc.). A mixture of 1.3 g of Witcolate ES-2 (30% solids, dodecyl ether sulfate, obtained from Witco Chemicals/Crompton Corporation, Middlebury, CT), 0.6 g of 1-hexanol, 6.4 g of de-ionized water, 1.5 g of AG8025 (Asahi Glass Company), 0.2 g of melamine resin (Aerotex 3730 from Cytec), and 0.02 g of catalyst (zinc nitrate) was prepared. This mixture wetted the expanded PTFE immediately. The coated ePTFE was placed in a solvent oven at 190°C for 3 min. The air permeability of the cured sample was measured (Gurley of 48.7 sec for 100 cm3). The sample was also determined to be oleophobic (oil rating of 8 on the coated side and 6 on the uncoated side).

**Example 9 (not falling within the scope of the invention)** In this example, a 5 mil thick, high molecular weight microporous polyethylene (Dewal Corporation) was rendered oleophobic and air permeable using surfactant and hexanol blends with fluoropolymers in accordance with the present invention. Specifically, a mixture of 1.3 g of Witcolate ES-2 (30% solids, dodecyl ether sulfate, obtained from Witco Chemicals/Crompton Corporation, Middlebury, CT), 0.6 g 1-hexanol, 5.1 g de-ionized water, and 3.0 g of AG8025 (Asahi Glass Company) was prepared. This mixture was observed to wet the microporous polyethylene membrane. The coated membrane was heated at 190°C for 2 min. The oleophobicity of the coated and uncoated sides was measured and was determined to be an oil rating of 7 for each side. A sample of the uncoated precursor polyethylene membrane had an oil rating of less than 1. The air permeability was also measured for the coated sample and the uncoated precursor. The coated sample had a Gurley (100 $cm^3$) measurement of 1.5 sec. The uncoated precursor polyethylene microporous membrane had a Gurley (100 $cm^3$) of 0.3 sec.

In the following examples, Examples 10-17, three types of ePTFE were used. The first two types of ePTFE were made according to the teachings of Branca et. al., US Patent No. 5,814,405. In the ePTFE designated Type 1, the membrane had an average mass of 4.4 $g/m^2$, a Gurley of 3.2 seconds, and a mean flow pore size of 0.21 microns. In the membrane designated Type 2, the membrane had an average mass of 4.7 $g/m^2$, a Gurley of 0.1 seconds, and a mean flow pore size of 4.5 microns. The final membrane, designated Type 3, was made according to the teachings of Gore, U.S. Patent No. 5,395,566 and had an average mass of 4.5 $g/m^2$, a Gurley of 0.6 seconds, and a mean flow pore size of 0.75 microns. These ePTFE membranes were used to demonstrate the wide range of mean flow pore size membranes that the instant invention is capable of filling with particulates.

## Examples 10-12

A mixture of 4.81 g of ZnO (Aldrich Chemical, St. Louis, MO) and 6.29 g of water was prepared. This mixture was not fluid (it was a dry paste). Since the mixture was not fluid, it could not be coated on ePTFE by placing a drop on ePTFE and spreading with a pipette bulb (to simulate typical application methods such as dip or kiss roll). A small amount of Aquatreat AR-540 (0.17 g) (AkzoNobel, Chicago, IL) was added, and the paste-like mixture became very fluid (water-like). This allowed the mixture to be sonicated (1 min at 1 setting with extended tip with a Misonix 3000 sonicator). Rhodapex ES-2 (AkzoNobel, Chicago, IL) (1.03 g) and 1-hexanol (0.46 g) were added and mixed to emulsify the hexanol. The mixture was still fluid. The mixture was a stable suspension. The mixture was then separately coated on Type 1 (Example 10), Type 2 (Example 11), and Type 3 (Example 12) ePTFE membranes by placing a drop of the mixture on one side of each ePTFE and spreading the mixture with essentially no pressure so that there was no surface excess. All of the ePTFEs were wetted by the mixture; significant penetration was observed. For Example 11, touching the non-coated side of the ePTFE showed that a high level of white slurry was present. The samples were dried for 3 min at 150°C. Scanning Electron Micrographs (SEMs) showed that ZnO particles fully filled the ePTFEs, as illustrated in Figure 1 for Example 11. Surprisingly, there was no surface excess and the distribution was uniform. Equally surprisingly, aerial mass measurements showed very high ZnO loadings. The aerial mass increased from 3.0 g/m2 to 60.7 g/m2 in Example 11. This represents a loading by weight of 95% ZnO and 5% ePTFE.

## Examples 13-15

A mixture of 39.99 g of water, 2.35 g of Barlox 10s (Lonza, Switzerland), 1.31 g of 1-hexanol, and 5.95 g of hydrophobic silica (Aerosil R9200, Evonix Industries AG, Hanau, Germany) was prepared. This mixture was stirred and sonicated for 2.5 min (Misonix 3000 Sonicator on setting of 3 with the extended tip). The mixture had significant foam, and it was slightly viscous. Then, Tamai 731A (Dow, Philadelphia, PA) (0.12 g) was added to 4.95 g of the above mixture. The Tamai 731A addition made the mixture very fluid (water-like) and eliminated all of the foam. This mixture was also coated on the Type 1, Type 2, and Type 3 ePTFEs as before to prepare Examples 13, 14, and 15 respectively. It was found to wet the substrates as fast as it could be spread. Furthermore, SEMs of Example 13 (Figure 2) showed high loading that was uniformly distributed through the thickness of the ePTFE. Aerial mass measurements showed high $SiO_2$ loadings. For Example 13, the aerial mass increased from 5.1 $g/m^2$ for the raw ePTFE to 8.1 g/m2 for the $SiO_2$ filled ePTFE. This represents a loading of 37% $SiO_2$ and 63% ePTFE.

## Example 16

The following mix was prepared (materials are listed in their order of addition): Raven 2500 Ultra Carbon Black (Columbian Chemicals Co., Marietta, GA) (4.85g), Barlox 12 Surfactant (Lonza, Switzerland) (1.09g), de-ionized water (12.66g), Solsperse 46000 Dispersant (Lubrizol, Sheffield Village, OH) (1.83g), and 1-hexanol (0.63g). The mix was shaken after the addition of the water and the dispersant to mix the materials, and the final mix was sonicated with an ultrasonic horn. After ultrasonication the mix was uniform and fluid with water-like viscosity. A small amount of the mix was pipetted onto one side of a Type 1 ePTFE membrane that was restrained in an embroidery hoop to maintain tension. The mix was spread using a PTFE roller so that essentially no excess remained on the surface. Good filling of the membrane was confirmed by touching the opposite side of the ePTFE and noting that the mix with a high concentration of black solids had penetrated all the way through the ePTFE. The samples were dried with a hair drier and then heat-treated in a hot-air convection oven at 280°C for 3 min. SEMs of both the heat-treated and non-heat-treated samples

showed that carbon was highly loaded and uniformly distributed through the thickness of the ePTFE.

**Example 17**

The following mix was prepared (materials are listed in their order of addition): Ludox TM-50 Colloidal Silica (Aldrich Chemical, St. Louis, MO) (14.05g of the 50% solids suspension), de-ionized water (3.93g), Rhodapex ES-2 (1.9g), and 1-hexanol (0.82g). The mixture was blended by gently shaking it by hand after the addition of the Rhodapex ES-2 and 1-hexanol, at which point it became slightly viscous and cloudy with low foam. After placing a drop of the solution onto Type 1 ePTFE, complete wetting of the surface was observed in 1-2 seconds. About 2g of mix was transferred onto one side of Type 1 ePTFE. The Type 1 ePTFE was restrained in a hoop to maintain tension. The mix was spread over the ePTFE surface with essentially no pressure and the entire wetted area of the ePTFE became transparent. The excess mix was removed, and the hoop was then placed in a hot-air convection oven at 150°C for 3 minutes to dry. The sample was dry and substantially transparent when removed from the oven. Aerial mass measurements showed high $SiO_2$ loadings, with the aerial mass increasing from 6.6 g/m2 for the raw ePTFE to 27.8 g/m2 for the $SiO_2$ imbibed ePTFE. This represents a loading of 76% $SiO_2$ and 24% ePTFE. An SEM (Figure 3) showed the silica was uniformly distributed through the thickness of the ePTFE.

## Claims

**1.** An aqueous mixture comprising
at least one water insoluble alcohol comprising a $C_5$-$C_{10}$ linear backbone,
at least one surfactant selected from anionic surfactants having a structure of $R(EO)_nOSO_3^-$ or $ROSO_3^-$ where R can be any organic chain, "O" is oxygen, "S" is sulfur, "EO" is ethylene oxide and n=>1, and nonionic surfactants having the structure $R(EO)_nOH$, where n=>1, said at least one surfactant forms an emulsion of said at least one water insoluble alcohol in water,
water insoluble inorganic particles comprising water insoluble compounds or elements selected from the group consisting of metals, carbon, metal oxides, metal hydroxides, metal nitrides, silicates, carbonates, sulfonates, phosphates, nitrates and mixtures thereof in a concentration of 10 wt % or higher based on the total weight of the emulsion, and
optionally a dispersant,
such that said aqueous mixture forms a fluid dispersion with said inorganic particles that wets an expanded polytetrafluoroethylene membrane sufficiently to uniformly fill at least a portion of the pores though the thickness of the expanded polytetrafluoroethylene membrane, the aqueous mixture having a surface tension of less than or equal to 0.03 N/m (30 dynes/cm) when applied on the expanded polytetrafluoroethylene membrane.

**2.** The aqueous mixture of claim 1, wherein said at least one water insoluble alcohol is present in an amount of up to 30% by weight of the aqueous mixture, particularly in an amount of up to 8% by weight of the aqueous mixture.

**3.** The aqueous mixture of claim 1, wherein said at least one surfactant is present in an amount of up to 15% by weight of the aqueous mixture.

**4.** The aqueous mixture of claim 1, wherein said water insoluble inorganic particles comprise elements in Group IA, IIA, IIIB, IVB, VB, VIB, VIIB, VIII, IB, IIB, IIIA, IVA, VA, and VIA of the periodic table; metal oxides or hydroxides; metal nitrides; silicates; carbonates; sulfonates; phosphates; nitrates; and mixtures thereof.

**5.** The aqueous mixture of claim 4, wherein said water insoluble inorganic particles comprise silica, or wherein said water insoluble inorganic particles comprise ZnO, or wherein said water insoluble inorganic particles comprise Carbon.

**6.** An aqueous mixture of claim 1
wherein said aqueous mixture wets said expanded polytetrafluoroethylene membrane in 10 seconds or less.

**7.** A method of filling a microporous low surface energy substrate comprising:

providing an aqueous mixture as claimed in any one of claims 1 to 6 and
applying the aqueous mixture to the surface of the microporous low surface energy substrate.

8. The method of claim 7, further comprising: applying mechanical energy to said aqueous mixture.

9. The method of claim 8 further comprising: heating the material to temperatures between 35 and 320 degrees Centigrade for a period of time.

10. An article comprising
a low surface energy microporous material; and
water insoluble inorganic particles that uniformly fill at least a portion of the pores of said microporous material, said article comprising up to 10% by weight surfactant based on the total weight of the article.

11. The article of claim 10, wherein said water insoluble inorganic particles comprise at most 96% of the article by weight, particularly
said inorganic particles comprise at most 75% of the article by weight, particularly
said inorganic particles comprise at most 50% of the article by weight, particularly
said inorganic particles comprise at most 30% of the article by weight, particularly
said inorganic particles comprise at most 20% of the article by weight, particularly
said inorganic particles comprise at most 10% of the article by weight, particularly
said inorganic particles comprise at most 5% of the article by weight.

12. The article of claim 10 wherein said low surface energy microporous material comprises expanded polytetrafluoroethylene.

## Patentansprüche

1. Wässrige Mischung, umfassend
zumindest einen wasserunlöslichen Alkohol, der ein lineares $C_5$-$C_{10}$-Rückgrat umfasst,
zumindest ein Tensid ausgewählt aus anionischen Tensiden mit einer Struktur von $R(EO)_nOSO_3^-$ oder $ROSO_3^-$, wobei R eine beliebige organische Kette sein kann, "O" Sauerstoff ist, "S" Schwefel ist, "EO" Ethylenoxid ist und n= >1, und nichtionischen Tensiden mit der Struktur $R(EO)_nOH$, wobei n= >1, wobei das zumindest eine Tensid eine Emulsion des zumindest einen wasserunlöslichen Alkohols in Wasser bildet,
wasserunlösliche anorganische Partikel, umfassend wasserunlösliche Verbindungen oder Elemente ausgewählt aus der Gruppe bestehend aus Metallen, Kohlenstoff, Metalloxiden, Metallhydroxiden, Metallnitriden, Silikaten, Carbonaten, Sulfonaten, Phosphaten, Nitraten und Mischungen davon in einer Konzentration von 10 Gew.-% oder höher basierend auf dem Gesamtgewicht der Emulsion, und
optional ein Dispergiermittel,
sodass die wässrige Mischung eine flüssige Dispersion mit den anorganischen Partikeln bildet, die eine expandierte Polytetrafluorethylenmembran ausreichend benetzt, um zumindest einen Teil der Poren gleichmäßig über die Dicke der expandierten Polytetrafluorethylenmembran zu füllen, wobei die wässrige Mischung eine Oberflächenspannung von weniger als oder gleich 0,03 N/m (30 dyn/cm) aufweist, wenn auf die expandierte Polytetrafluorethylenmembran aufgebracht.

2. Wässrige Mischung nach Anspruch 1, wobei der zumindest eine wasserunlösliche Alkohol in einer Menge von bis zu 30 Gew.-% der wässrigen Mischung vorhanden ist, insbesondere in einer Menge von bis zu 8 Gew.-% der wässrigen Mischung.

3. Wässrige Mischung nach Anspruch 1, wobei das zumindest eine Tensid in einer Menge von bis zu 15 Gew.-% der wässrigen Mischung vorhanden ist.

4. Wässrige Mischung nach Anspruch 1, wobei die wasserunlöslichen anorganischen Partikel Elemente der Gruppe IA, IIA, IIIB, IVB, VB, VIB, VIIB, VIII, IB, IIB, IIIA, IVA, VA und VIA des Periodensystems; Metalloxide oder -hydroxide; Metallnitride; Silikate; Carbonate; Sulfonate; Phosphate; Nitrate und Mischungen davon umfassen.

5. Wässrige Mischung nach Anspruch 4, wobei die wasserunlöslichen anorganischen Partikel Siliciumdioxid umfassen oder wobei die wasserunlöslichen anorganischen Partikel ZnO umfassen oder wobei die wasserunlöslichen anorganischen Partikel Kohlenstoff umfassen.

6. Wässrige Mischung nach Anspruch 1,
wobei die wässrige Mischung die expandierte Polytetrafluorethylenmembran in 10 Sekunden oder weniger benetzt.

7. Verfahren zum Füllen eines mikroporösen Substrats mit niedriger Oberflächenenergie, umfassend:

   Bereitstellen einer wässrigen Mischung nach einem der Ansprüche 1 bis 6, und
   Aufbringen der wässrigen Mischung auf die Oberfläche des mikroporösen Substrats mit niedriger Oberflächenenergie.

8. Verfahren nach Anspruch 7, ferner umfassend: Aufbringen von mechanischer Energie auf die wässrige Mischung.

9. Verfahren nach Anspruch 8, ferner umfassend:
Erhitzen des Materials auf Temperaturen zwischen 35 und 320 Grad Celsius für einen Zeitraum.

10. Artikel, umfassend
ein mikroporöses Material mit niedriger Oberflächenenergie; und
wasserunlösliche anorganische Partikel, die zumindest einen Teil der Poren des mikroporösen Materials gleichmäßig füllen, wobei der Artikel bis zu 10 Gew.-% Tensid umfasst, basierend auf dem Gesamtgewicht des Artikels.

11. Artikel nach Anspruch 10, wobei die wasserunlöslichen anorganischen Partikel höchstens 96 Gew.-% des Artikels umfassen, insbesondere
die anorganischen Partikel höchstens 75 Gew.-% des Artikels umfassen, insbesondere
die anorganischen Partikel höchstens 50 Gew.-% des Artikels umfassen, insbesondere
die anorganischen Partikel höchstens 30 Gew.-% des Artikels umfassen, insbesondere
die anorganischen Partikel höchstens 20 Gew.-% des Artikels umfassen, insbesondere
die anorganischen Partikel höchstens 10 Gew.-% des Artikels umfassen, insbesondere
die anorganischen Partikel höchstens 5 Gew.-% des Artikels umfassen.

12. Artikel nach Anspruch 10, wobei das mikroporöse Material mit niedriger Oberflächenenergie expandiertes Polytetrafluorethylen umfasst.

**Revendications**

1. Mélange aqueux comprenant
au moins un alcool insoluble dans l'eau comprenant un squelette linéaire en $C_5$ à $C_{10}$,
au moins un tensioactif choisi parmi les tensioactifs anioniques présentant une structure de $R(EO)_nOSO_3^-$ ou $ROSO_3^-$ où R peut être toute chaîne organique, «O» représente un atome d'oxygène, « S » représente un atome de soufre, « EO » représente un groupe oxyde d'éthylène et n=>1, et les tensioactifs non-ioniques présentant la structure $R(EO)_nOH$, où n=>1, ledit au moins un tensioactif formant une émulsion dans l'eau dudit au moins un alcool insoluble dans l'eau,
des particules inorganiques insolubles dans l'eau comprenant des composés ou éléments insolubles dans l'eau choisis dans le groupe constitué par les métaux, le carbone, les oxydes métalliques, les hydroxydes métalliques, les nitrures métalliques, les silicates, les carbonates, les sulfonates, les phosphates, les nitrates et les mélanges de ceux-ci en une concentration supérieure ou égale à 10 % par rapport au poids total de l'émulsion, et
éventuellement un dispersant,
de sorte que ledit mélange aqueux forme une dispersion fluide avec lesdites particules inorganiques qui mouillent une membrane en polytétrafluoroéthylène expansé suffisamment pour remplir uniformément au moins une partie des pores malgré l'épaisseur de la membrane en polytétrafluoroéthylène expansé, le mélange aqueux présentant une tension superficielle inférieure ou égale à 0,03 N/m (30 dynes/cm) lorsqu'il est appliqué sur la membrane en polytétrafluoroéthylène expansé.

2. Mélange aqueux selon la revendication 1, ledit au moins un alcool insoluble dans l'eau étant présent en une quantité représentant jusqu'à 30 % en poids du mélange aqueux, en particulier en une quantité représentant jusqu'à 8 % en poids du mélange aqueux.

3. Mélange aqueux selon la revendication 1, ledit au moins tensioactif étant présent en une quantité représentant jusqu'à 15 % en poids du mélange aqueux.

4. Mélange aqueux selon la revendication 1, lesdites particules inorganiques insolubles dans l'eau comprenant des éléments dans les groupes IA, IIA, IIIB, IVB, VB, VIB, VIIB, VIII, IB, IIB, IIIA, IVA, VA et VIA du tableau périodique ; des oxydes ou des hydroxydes métalliques ; des nitrures métalliques ; des silicates ; des carbonates ; des sulfonates ; des phosphates ; des nitrates ; et des mélanges de ceux-ci.

5. Mélange aqueux selon la revendication 4, lesdites particules inorganiques insolubles dans l'eau comprenant de la silice, ou lesdites particules inorganiques insolubles dans l'eau comprenant du ZnO, ou lesdites particules inorganiques insolubles dans l'eau comprenant du carbone.

6. Mélange aqueux selon la revendication 1,
ledit mélange aqueux mouillant ladite membrane en polytétrafluoroéthylène expansé en 10 secondes ou moins.

7. Procédé de remplissage d'un substrat microporeux à faible énergie de surface comprenant :

la fourniture d'un mélange aqueux selon l'une quelconque des revendications 1 à 6 et
l'application du mélange aqueux sur la surface du substrat microporeux à faible énergie de surface.

8. Procédé selon la revendication 7, comprenant en outre :
l'application d'une énergie mécanique sur ledit mélange aqueux.

9. Procédé selon la revendication 8, comprenant en outre :
le chauffage du matériau à des températures comprises entre 35 et 320 degrés centigrades pendant un laps de temps.

10. Article comprenant
un matériau microporeux à faible énergie de surface ; et
des particules inorganiques insolubles dans l'eau qui remplissent uniformément au moins une partie des pores dudit matériau microporeux, ledit article comprenant jusqu'à 10 % en poids de tensioactif par rapport au poids total de l'article.

11. Article selon la revendication 10, lesdites particules inorganiques insolubles dans l'eau constituant au plus 96 % de l'article en poids, en particulier
lesdites particules inorganiques constituant au plus 75 % de l'article en poids, en particulier
lesdites particules inorganiques constituant au plus 50 % de l'article en poids, en particulier
lesdites particules inorganiques constituant au plus 30 % de l'article en poids, en particulier
lesdites particules inorganiques constituant au plus 20 % de l'article en poids, en particulier
lesdites particules inorganiques constituant au plus 10 % de l'article en poids, en particulier
lesdites particules inorganiques constituant au plus 5 % de l'article en poids.

12. Article selon la revendication 10, ledit matériau microporeux à faible énergie de surface comprenant du polytétrafluoroéthylène expansé.

FIGURE 1

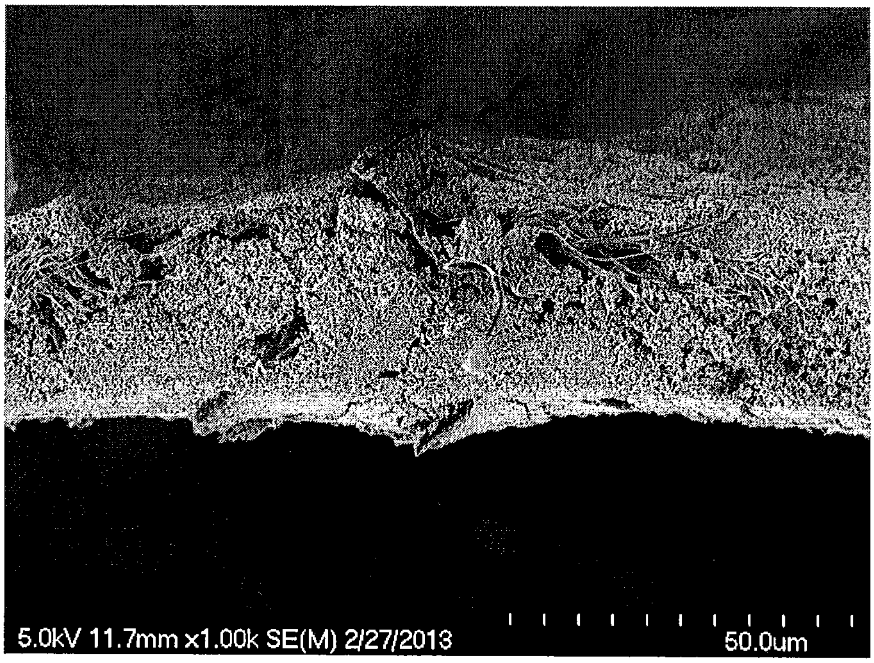

FIGURE 2

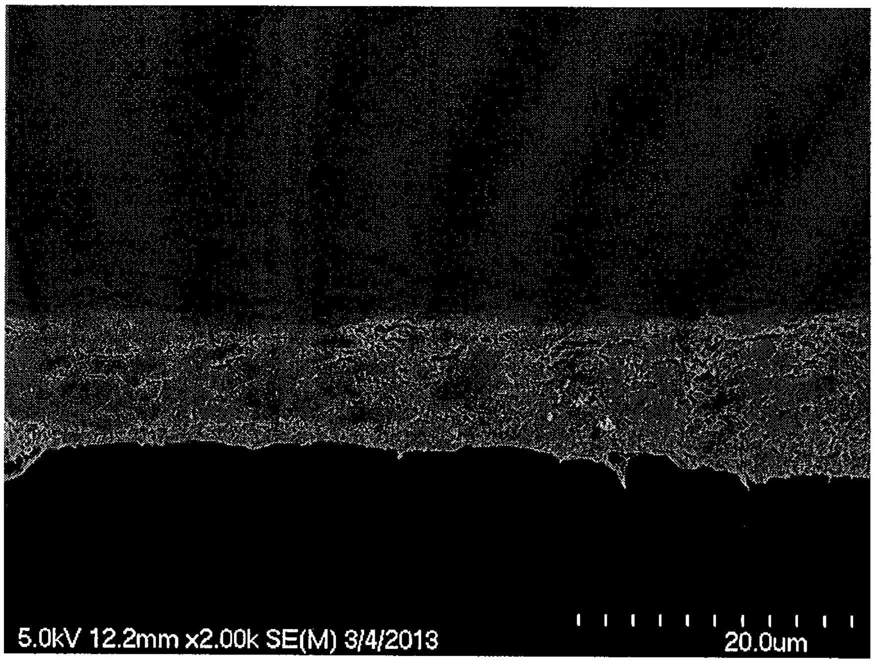

FIGURE 3

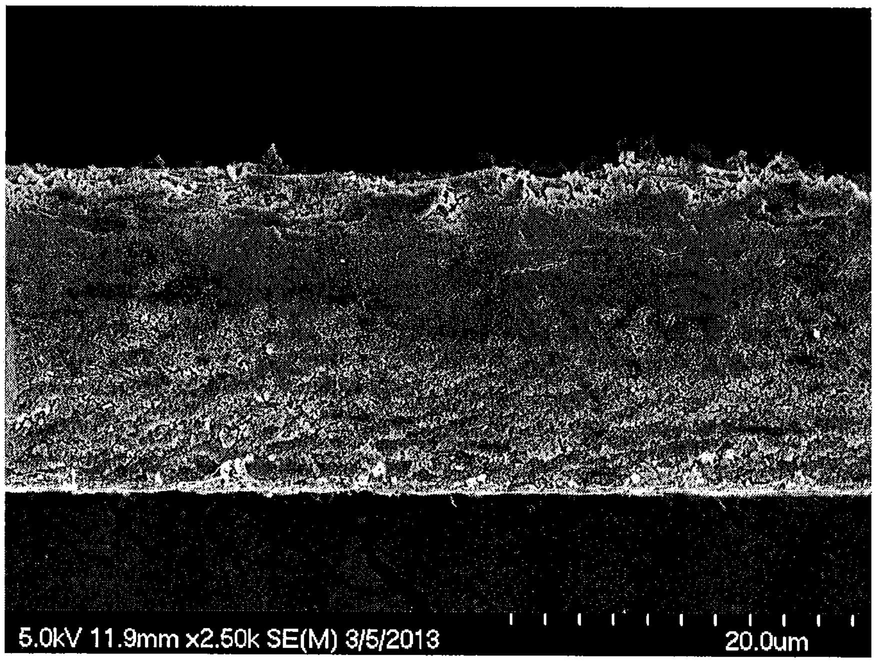

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5952004 A, Rudnic
- EP 0581168 A
- WO 9210532 A
- EP 0561875 A
- US 6228477 B
- US 20060270744 A, Freese
- WO 2006127937 A, Freese
- WO 2012135895 A, R.J. Roberts
- US 5460872 A, Wu
- US 6676993 B2
- EP 0615779 A
- US 5539072 A
- US 5814405 A
- US 5395566 A